# EUROPEAN PATENT APPLICATION

(11) **EP 4 806 333 A1**
(43) Date of publication of application: **16.09.2026**
(21) Application number: 25220555.4
(22) Date of filing: 03.12.2025
(51) Int. Cl.: A61B 1/00, A61B 1/04, A61B 5/00

(54) **IMAGING CATHETER**

(30) Priority: 04.12.2024 KR 20240178079
(71) Applicant: Dotter Inc., Incheon 22004 (KR)
(72) Inventor: KIM, Hyungil, 22001 Incheon (KR); LEE, Min Woo, 21986 Incheon (KR); KO, Kyungjoon, 04738 Seoul (KR); HAN, Sohyeon, 10360 Gyeonggi-do (KR); LEE, Juyeon, 07740 Seoul (KR)
(74) Representative: Dörries, Hans Ulrich

(57) **Abstract**

According to one aspect of the present disclosure for achieving the above-described object, a multimodal imaging catheter inserted into the body to acquire information of an internal body tissue for achieving the above-described object is disclosed. The multimodal imaging catheter may include an imaging core including a double clad fiber and a focusing device, the imaging core transfers light to the internal body tissue and collects information within the internal body tissue; and an imaging sheath including the imaging core therein to protect the imaging core from an external environment and to separate the internal body tissue and the imaging core during an imaging operation of the multimodal imaging catheter for the internal body tissue. Also the imaging sheath includes a plurality of tubes coupled along a longitudinal direction of the multimodal imaging catheter, a first tube corresponding to an imaging window region through which light generated according to a rotation of the focusing device passes among a plurality of tubes of the imaging sheath is made of a first material in which a maximum intensity of autofluorescence when excited light is irradiated is smaller than a standard deviation of background noise when excited light is not irradiated, in order to reduce autofluorescence generated by the imaging sheath itself, a second tube corresponding to a distal tip region from which the light is emitted to the internal body tissue among a plurality of tubes of the imaging sheath is made of a second material in which a maximum intensity of autofluorescence when excited light is irradiated is larger than a standard deviation of background noise when excited light is not irradiated, and when the multimodal imaging catheter is inserted into a body, the first tube in the imaging sheath is located proximal to the internal body tissue, and the second tube may be located distal to the internal body tissue.

## Description

### CROSS-REFERENCE TO RELATED APPLICATIONS

This application claims priority to and the benefit of Korean Patent Application No. 10-2024-0178079 filed in the Korean Intellectual Property Office on December 04, 2024, the entire contents of which are incorporated herein by reference.

### TECHNICAL FIELD

This disclosure relates to a catheter for measuring fluorescence lifetime or a multimodal imaging catheter.

### BACKGROUND ART

There exist imaging technologies for diagnosing the presence or absence of a disease in a tissue and the progression degree of the disease using autofluorescence characteristics generated in internal body tissue.

Among such imaging technologies are intravascular imaging technologies for diagnosing coronary artery disease, endoscopy-related imaging technologies for diagnosing gastrointestinal tract related diseases, and otoscope imaging technologies for diagnosing diseases inside an ear.

Specifically, the imaging technologies may include optical coherence tomography (OCT), fluorescence lifetime imaging (FLIm), near infrared fluorescence imaging (NIRF), near infrared autofluorescence (NIRAF), near infrared spectroscopy (NIRS), and intravascular ultrasound (IVUS), and the like.

The listed technologies may be used individually or the listed technologies may be applicable to multimodal imaging technologies in which two or more of them are used simultaneously.

Although an imaging catheter system for implementing the above-described technologies exists, an issue may arise in that unwanted signals are acquired due to autofluorescence generated from the catheter system itself.

Korean Registered Patent Publication No. KR 10-1658447, can be considered prior art.

### SUMMARY OF THE INVENTION

The present disclosure has been contrived in response to the above-described background art, and has been made in an effort to provide a catheter and a method for efficiently removing noise acquired during a process of measuring fluorescence lifetime and for calibrating fluorescence intensity and fluorescence lifetime acquired by the catheter.

Technical objects of the present disclosure are not restricted to the technical object mentioned as above. Other unmentioned technical objects will be apparently appreciated by those skilled in the art by referencing the following description.

According to one aspect of the present disclosure for achieving the above-described object, a multimodal imaging catheter inserted into the body to acquire information of an internal body tissue for achieving the above-described object is disclosed. The multimodal imaging catheter may include an imaging core including a double clad fiber and a focusing device, the imaging core transfers light to the internal body tissue and collects information within the internal body tissue; and an imaging sheath including the imaging core therein to protect the imaging core from an external environment and to separate the internal body tissue and the imaging core during an imaging operation of the multimodal imaging catheter for the internal body tissue. Also the imaging sheath includes a plurality of tubes coupled along a longitudinal direction of the multimodal imaging catheter, a first tube corresponding to an imaging window region through which light generated according to a rotation of the focusing device passes among a plurality of tubes of the imaging sheath is made of a first material in which a maximum intensity of autofluorescence when excited light is irradiated is smaller than a standard deviation of background noise when excited light is not irradiated, in order to reduce autofluorescence generated by the imaging sheath itself, a second tube corresponding to a distal tip region from which the light is emitted to the internal body tissue among a plurality of tubes of the imaging sheath is made of a second material in which a maximum intensity of autofluorescence when excited light is irradiated is larger than a standard deviation of background noise when excited light is not irradiated, and when the multimodal imaging catheter is inserted into a body, the first tube in the imaging sheath is located proximal to the internal body tissue, and the second tube may be located distal to the internal body tissue.

In one embodiment, the first material may be enhanced fluorinated ethylene propylene.

In one embodiment, the second material may be a copolymer composed of two blocks of polyether and polyamide.

In one embodiment, the multimodal imaging catheter may be a catheter in which the first tube and the second tube are coupled in a manner in which at least a part of an outer surface of the second tube is inserted into an inner surface of the first tube such that a partial region of the first tube and the second tube is in contact, and a mandrel for maintaining a diameter of the inner surface of the first tube is inserted into the coupled first tube and second tube during a coupling process of the first tube and the second tube.

In one embodiment, the multimodal imaging catheter may be disposed at a position corresponding to the partial region where the first tube and the second tube are in contact, wherein a heat shrink tube that externally surrounds the first tube and the second tube during a coupling process of the first tube and the second tube. Also the heat shrink tube may couple the first tube and the second tube such that the coupling between the first tube and the second tube can withstand a force equal to or greater than a specific reference size by physically changing its shape by hot air or cold air.

In one embodiment, the focusing device of the imaging core may be positioned in the imaging window region corresponding to the first tube during the imaging operation of the multimodal imaging catheter for the internal body tissue.

In one embodiment, the multimodal imaging catheter may calculate a calibration value of the multimodal imaging catheter using a predefined fluorescence lifetime value of an autofluorescence of the second tube before the imaging operation for the internal body tissue.

In one embodiment, the focusing device of the imaging core may be moved from a first position in the imaging window region corresponding to the first tube to a second position in the distal tip region corresponding to the second tube to calculate the calibration value of the multimodal imaging catheter.

In one embodiment, the calibration value may include a first calibration value calculated based on first fluorescence intensity information corresponding to an intensity of the autofluorescence acquired while the focusing device is provided at a first position of the imaging window region and second fluorescence intensity information corresponding to an intensity of the autofluorescence acquired while the focusing device is provided at a second position of the distal tip region.

In one embodiment, the calibration value may include a second calibration value calculated based on first fluorescence lifetime information corresponding to a lifetime of the autofluorescence acquired while the focusing device is provided at a first position of the imaging window region and second fluorescence lifetime information corresponding to a lifetime of fluorescence acquired while the focusing device is provided at a second position of the distal tip region.

In one embodiment, the multimodal imaging catheter may calibrate fluorescence intensity and fluorescence lifetime acquired by the multimodal imaging catheter by applying the calibration value to fluorescence information acquired while the focusing device is provided at the first position during the imaging operation of the multimodal imaging catheter for the internal body tissue.

A fluorescence lifetime imaging catheter inserted into a body to measure a fluorescence lifetime of an internal body tissue according to another embodiment of the present disclosure is disclosed. In one embodiment, the fluorescence lifetime imaging catheter may include an imaging core including an optical fiber and a focusing device, the imaging core transfers light to the internal body tissue and collects fluorescence emitted by a fluorescent substance within the internal body tissue; and an imaging sheath including the imaging core therein to protect the imaging core from an external environment and to separate the internal body tissue and the imaging core during an imaging operation of the fluorescence lifetime imaging catheter for the internal body tissue. Also the imaging sheath includes a plurality of tubes coupled along a longitudinal direction of the fluorescence lifetime imaging catheter, a first tube corresponding to an imaging window region through which light generated according to a rotation of the focusing device passes among a plurality of tubes of the imaging sheath is made of a first material in which a maximum intensity of autofluorescence when excited light is irradiated is smaller than a standard deviation of background noise when excited light is not irradiated, in order to reduce autofluorescence generated by the imaging sheath itself, a second tube corresponding to a distal tip region from which the light is emitted to the internal body tissue among a plurality of tubes of the imaging sheath is made of a second material in which a maximum intensity of autofluorescence when excited light is irradiated is larger than a standard deviation of background noise when excited light is not irradiated, and when the fluorescence lifetime imaging catheter is inserted into a body, the first tube in the imaging sheath is located proximal to the internal body tissue, and the second tube may be located distal to the internal body tissue.

In one embodiment, the first material may be enhanced fluorinated ethylene propylene.

In one embodiment, the second material may be a copolymer composed of two blocks of polyether and polyamide.

In one embodiment, the fluorescence lifetime imaging catheter may be a catheter in which the first tube and the second tube are coupled in a manner in which at least a part of an outer surface of the second tube is inserted into an inner surface of the first tube such that a partial region of the first tube and the second tube is in contact, and a mandrel for maintaining a diameter of the inner surface of the first tube is inserted into the coupled first tube and second tube during a coupling process of the first tube and the second tube.

In one embodiment, the fluorescence lifetime imaging catheter may be disposed at a position corresponding to the partial region where the first tube and the second tube are in contact, wherein a heat shrink tube that externally surrounds the first tube and the second tube during a coupling process of the first tube and the second tube. Also the heat shrink tube may couple the first tube and the second tube such that the coupling between the first tube and the second tube can withstand a force equal to or greater than a specific reference size by physically changing its shape by hot air or cold air.

In one embodiment, the focusing device of the imaging core may be positioned in the imaging window region corresponding to the first tube during the imaging operation of the fluorescence lifetime imaging catheter for the internal body tissue.

In one embodiment, the fluorescence lifetime imaging catheter may calculate a calibration value of the fluorescence lifetime imaging catheter using a predefined fluorescence lifetime value of the autofluorescence of the second tube before the imaging operation for the internal body tissue.

In one embodiment, the focusing device of the imaging core may be moved from a first position in the imaging window region corresponding to the first tube to a second position in the distal tip region corresponding to the second tube to calculate the calibration value of the fluorescence lifetime imaging catheter.

In one embodiment, the calibration value may include a first calibration value calculated based on first fluorescence intensity information corresponding to the intensity of the fluorescence acquired while the focusing device is provided at a first position of the imaging window region and second fluorescence intensity information corresponding to the intensity of the fluorescence acquired while the focusing device is provided at a second position of the distal tip region.

In one embodiment, the calibration value may include a second calibration value calculated based on first fluorescence lifetime information corresponding to a lifetime of the autofluorescence acquired while the focusing device is provided at a first position of the imaging window region and second fluorescence lifetime information corresponding to a lifetime of the autofluorescence acquired while the focusing device is provided at a second position of the distal tip region.

In one embodiment, the fluorescence lifetime imaging catheter may calibrate fluorescence intensity and fluorescence lifetime acquired by the fluorescence lifetime imaging catheter by applying the calibration value to fluorescence information acquired while the focusing device is provided at the first position during the imaging operation of the fluorescence lifetime imaging catheter for the internal body tissue.

Technical solutions which can be acquired in the present disclosure are not limited to the technical solutions mentioned above, and other unmentioned technical solutions will be clearly understood by those skilled in the art from the following description.

According to one embodiment of the present disclosure, a catheter may efficiently remove noise acquired during a process of measuring fluorescence lifetime and may calibrate fluorescence intensity and fluorescence lifetime.

Effects which can be acquired in the present disclosure are not limited to the aforementioned effects and other unmentioned effects will be clearly understood by those skilled in the art from the following description.

### BRIEF DESCRIPTION OF THE DRAWINGS

Various aspects are now described with reference to the drawings and like reference numerals are generally used to designate like elements. In the following embodiments, for purposes of explanation, numerous specific detailed matters are presented to provide a comprehensive understanding of one or more aspects. However, it will be apparent that the aspect(s) can be executed without the specific detailed matters. In other examples, known structures and apparatuses are illustrated in a block diagram form in order to facilitate description of one or more aspects.
**FIG.** 1 is a diagram for explaining a fluorescence lifetime imaging catheter according to some embodiments of the present disclosure.
**FIG.** 2 is a flowchart for explaining a method for coupling some configurations of a fluorescence lifetime imaging catheter according to some embodiments of the present disclosure.
**FIG.** 3 is a diagram for explaining a method for coupling some configurations of a fluorescence lifetime imaging catheter according to some embodiments of the present disclosure.
**FIG.** 4 is a flowchart for explaining a method for calculating a calibration value using a fluorescence lifetime imaging catheter according to some embodiments of the present disclosure.
**FIG.** 5 is a diagram for explaining a method for calculating a calibration value using a fluorescence lifetime imaging catheter according to some embodiments of the present disclosure.
**FIG.** 6 is a diagram for exemplarily explaining a result when imaging is performed using a fluorescence lifetime imaging catheter according to some embodiments of the present disclosure.
**FIG.** 7 illustrates a general schematic diagram for an exemplary computing environment in which embodiments of the present disclosure may be implemented.

### DETAILED DESCRIPTION

Various embodiments and/or aspects are now disclosed with reference to the drawings. For the purpose of explanation, numerous specific details are set forth in the following description to provide a thorough understanding of one or more aspects. However, it will also be perceptible to those skilled in the art to which this disclosure pertains that such aspect(s) may be implemented without these specific details. The subsequent description and the accompanying drawings detail specific exemplary aspects of one or more aspects. However, these aspects are exemplary and some of the various methods in the principles of the various aspects may be utilized, and the descriptions set forth are intended to include all of such aspects and their equivalents. Specifically, "embodiment," "example," "aspect," "illustration," and the like, as used herein, may not be interpreted as meaning that any described aspect or design is better or more advantageous than other aspects or designs.

Hereinafter, the same or similar constituent elements regardless of reference numerals are assigned the same reference numerals, and a redundant description thereof is omitted. In addition, when it is determined that a detailed description of related known technology may unnecessarily obscure the subject matter of the embodiment disclosed herein in describing the embodiment disclosed in this specification, the detailed description thereof is omitted. In addition, the accompanying drawings are only for allowing easy understanding of the embodiment disclosed in this specification, and the technical idea disclosed in this specification is not limited by the accompanying drawings.

Although first, second, etc., are used to describe various elements or components, it is of course understood that these elements or components are not limited by these terms. These terms are only used to distinguish one element or component from another element or component. Therefore, a first element or component mentioned below may be a second element or component within the technical idea of the present invention.

Unless otherwise defined, all terms (including technical and scientific terms) used herein may be used with the meaning commonly understood by one of ordinary skill in the art to which the present invention belongs. Also, terms defined in generally used dictionaries are not interpreted ideally or excessively unless they are clearly and specifically defined.

Furthermore, the term "or" is intended to mean an inclusive "or" rather than an exclusive "or". That is, unless otherwise specified or clearly not the case from the context, "X utilizes A or B" is intended to mean one of the natural inclusive permutations. That is, if X utilizes A; X utilizes B; or X utilizes both A and B, "X utilizes A or B" may apply to any of these cases. In addition, the term "and/or" as used herein should be understood as referring to and including any and all possible combinations of one or more of the associated listed items.

Also, the terms "comprising" and/or "comprises" are understood to mean the presence of the stated feature and/or component, but do not exclude the presence or addition of one or more other features, components, and/or groups thereof. In addition, unless otherwise specified or clearly indicated by the context to denote the singular form, the singular in the present specification and claims should generally be interpreted as meaning "one or more".

When a constituent element is referred to as being "connected" or "coupled" to another constituent element, it may be directly connected or coupled to the other constituent element, but it is to be understood that an intervening constituent element may be present. Conversely, when a constituent element is referred to as being "directly connected" or "directly coupled" to another constituent element, it is to be understood that no intervening constituent element is present.

The suffixes "module" and "unit" for constituent elements used in the following description are merely given for the sake of ease in writing the specification or are used interchangeably, and do not inherently have mutually distinguishable meanings or roles.

The objects and effects of the present disclosure, and the technical configurations for achieving them, will become clear by referring to the embodiments detailed below with the accompanying drawings. In describing the present disclosure, when it is determined that a detailed description of known functions or configurations may unnecessarily obscure the subject matter of the present disclosure, the detailed description thereof will be omitted. Also the terms described below are terms defined in consideration of the functions in the present disclosure, and these may differ according to a user's, operator's intention, or custom.

However, the present disclosure is not limited to the embodiments disclosed below but may be embodied in various different forms. These embodiments are merely provided to complete the disclosure and to fully inform those skilled in the art to which the present disclosure pertains of the scope of the disclosure, and the present disclosure is defined only by the scope of the claims. Therefore, the definition should be based on the contents throughout this specification.

In the present disclosure, the fluorescence lifetime imaging catheter may be a device that emits a wavelength such as light or sound waves to the outside of the catheter and detects the fluorescence lifetime of the internal body tissue using the wavelength that is reflected by the internal body tissue and re-enters the inside of the catheter. For example, the fluorescence lifetime imaging catheter is a device for evaluating and monitoring the state of biological tissue by utilizing optical technology, and can check the metabolic state and pathological state of the internal body tissue through an image. For example, the fluorescence lifetime imaging catheter may evaluate the characteristics of the internal body tissue by measuring a lifetime of a fluorescence signal reflected from the body tissue.

In the present disclosure, the multimodal imaging catheter may refer to a catheter that processes several types of medical images. In one embodiment, the multimodal imaging catheter may refer to a catheter that combines various image types or imaging technologies such as ultrasound, optical coherence tomography (OCT), and fluorescence imaging. In one embodiment, an imaging core of the multimodal imaging catheter for collecting information within the body (for example, the information may correspond to a wavelength, and may include a wavelength such as ultrasound, light, etc.) by transferring light to the internal body tissue may be configured with an optical fiber. For example, the optical fiber may be a double clad fiber.

In the present disclosure, the optical fiber may include a single mode fiber, a multimode fiber, and a double clad fiber. Also the double clad fiber may be a core of Germanium-doped silica material having a highest refractive index, a first cladding that is of silica material having a second highest refractive index and surrounds an outer periphery of the core, and a second cladding that surrounds the outer periphery of the first cladding having a third highest refractive index. Also, the imaging sheath may include a plurality of tubes coupled along a longitudinal direction of the fluorescence lifetime imaging catheter. A first tube corresponding to an imaging window region through which light generated according to a rotation of the focusing device passes among a plurality of tubes of the imaging sheath may be made of a first material in which a maximum intensity of autofluorescence when excited light is irradiated is smaller than a standard deviation of background noise when excited light is not irradiated, in order to reduce autofluorescence generated by the imaging sheath itself. A second tube corresponding to a distal tip region from which the light is emitted to the internal body tissue among a plurality of tubes of the imaging sheath may be made of a second material in which a maximum intensity of autofluorescence when excited light is irradiated is larger than a standard deviation of background noise when excited light is not irradiated.

In the present disclosure, the double clad fiber may be an optical fiber in a cladding-added form in a general optical fiber composed of a core through which a wavelength such as light is transmitted and a cladding surrounding the core. In one embodiment, the double clad fiber may be composed of a first cladding surrounding the core and a second cladding surrounding the first cladding. The double clad fiber can transfer information within the tissue more efficiently by receiving less external influence through two claddings.

In the present disclosure, light or sound waves are transmitted through the imaging core, and an imaging sheath for protecting the imaging core may be provided in a form surrounding an exterior of the imaging core.

The imaging core often acquires a tubular tissue image while rotating and moving in a longitudinal direction, but depending on the purpose, both rotation and longitudinal movement may not be performed, or only one of them may be performed.

The imaging sheath may be a tube that serves to separate and protect a tubular body tissue and the imaging core, and may be composed of a combination of several types of plastic tubes. The imaging sheath may not induce a specific motion such as rotation or longitudinal movement.

The imaging sheath may be configured in various forms according to the characteristics of the imaging technology and the tissue to be observed, and may include an area for transmitting light and/or sound waves.

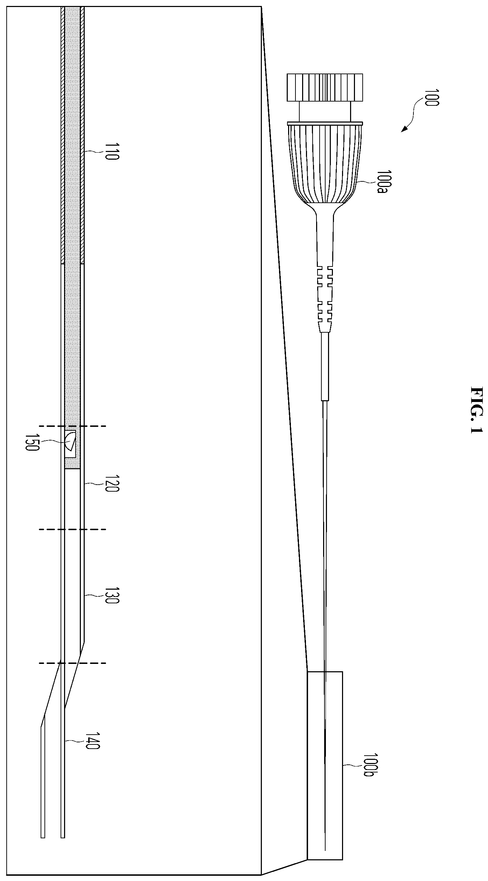

FIG. 1 is a diagram for explaining an imaging catheter according to some embodiments of the present disclosure. The imaging catheter in the present disclosure may be used in a comprehensive sense to include the multimodal imaging catheter and the fluorescence lifetime imaging catheter. Hereinafter, for convenience of description, the fluorescence lifetime imaging catheter is exemplarily described, but the fluorescence lifetime imaging catheter may be used in a sense including the multimodal imaging catheter.

Referring to FIG. 1, some configurations of a fluorescence lifetime imaging catheter 100 inserted into the body to measure a fluorescence lifetime of an internal body tissue are disclosed. In one embodiment, the fluorescence lifetime imaging catheter 100 may include an insertion unit 100b inserted into the body to collect fluorescence from the body tissue, and a connection unit 100a connected to a device for transferring rotational force after the insertion unit 100b is inserted into the body.

In one embodiment, the insertion unit 100b of the fluorescence lifetime imaging catheter 100 may include a shaft tube 110 corresponding to a main body of the insertion unit 100b, an imaging window region 120 for collecting fluorescence emitted by a fluorescent substance within the body tissue, a distal tip 130 for the insertion unit 100b to be inserted into the body, and a monorail 140 corresponding to a guide for the distal tip 130 to be moved, in order from the one closest to the connection unit 100a.

In one embodiment, the fluorescence lifetime imaging catheter 100 may include a focusing device 150.

In the present disclosure, the focusing device 150 may be a tool for concentrating light inside the fluorescence lifetime imaging catheter 100. For example, the focusing device 150 may be a lens or a concave mirror.

In one embodiment, the focusing device 150 may be included in an internal region corresponding to the imaging window region 120 and/or the distal tip 130 inside the fluorescence lifetime imaging catheter 100.

In one embodiment, the fluorescence lifetime imaging catheter 100 may include an imaging core that transfers light to the internal body tissue and collects fluorescence emitted by a fluorescent substance within the internal body tissue. In one embodiment, the imaging core may be included inside the insertion unit 100b, and may include an optical fiber and the focusing device 150. Also, the optical fiber may include a single mode fiber, a multimode fiber, and a double clad fiber. Also, the double clad fiber may be a core of Germanium-doped silica material having a highest refractive index, a first cladding that is of silica material having a second highest refractive index and surrounds an outer periphery of the core, and a second cladding that surrounds the outer periphery of the first cladding having a third highest refractive index.

In one embodiment, during an imaging operation of the fluorescence lifetime imaging catheter 100 for the internal body tissue, the focusing device 150 of the imaging core may be positioned in the imaging window region 120 corresponding to the first tube.

In one embodiment, the fluorescence lifetime imaging catheter 100 may include an imaging sheath that includes the imaging core therein to protect the imaging core from an external environment and to separate the internal body tissue and the imaging core during an imaging operation of the fluorescence lifetime imaging catheter 100 for the internal body tissue.

In one embodiment, a plurality of tubes coupled along a longitudinal direction of the fluorescence lifetime imaging catheter 100 may be included.

In one embodiment, the plurality of tubes may include a first tube corresponding to the imaging window region 120 through which light generated according to a rotation of the focusing device 150 passes among a plurality of tubes of the imaging sheath. For example, the first tube may form a part corresponding to the imaging window region 120 in the sheath of the catheter 100. For example, a second tube may form a part corresponding to the distal tip 130 in the sheath of the catheter 100.

In one embodiment, the first tube may be made of a first material in which a maximum intensity of autofluorescence when excited light is irradiated is smaller than a standard deviation of background noise when excited light is not irradiated, in order to reduce autofluorescence generated by the imaging sheath itself. For example, the first material may be enhanced fluorinated ethylene propylene. Enhanced fluorinated ethylene propylene may be a material made of ethylene, tetrafluoroethylene (TFE), and hexafluoropropylene (HFP).

In one embodiment, background noise may refer to information that is unnecessarily acquired and not used in the present invention. The background noise may distort necessary information to be used in the present invention. This background noise may include noise such as noise generated by the catheter (e.g., irregularly outputted light, noise due to temperature and humidity, etc.), light generated from an external environment, noise, and vibration.

In one embodiment, the standard deviation of background noise may be an index that quantitatively expresses variability and intensity of the background noise. The standard deviation of background noise may be a standard deviation derived from an actual value and an average value of the background noise. For example, the standard deviation of background noise may be a value included in a range of 0.5 to 2.0 dB.

In one embodiment, the first material may have an advantage in that there is almost no use restriction due to a wavelength because it has a high transmittance for a wide wavelength region (ultraviolet-visible light-near infrared). In addition, the first material may reduce the intensity of noise caused by the imaging sheath of the catheter itself. In addition, since the first material has a higher stiffness than a sheath material used in conventional intravascular imaging catheters, there is an advantage of excellent pushability (e.g., intravascular insertability). In addition, the first material can replace the sheath of the conventional intravascular imaging catheter because the difference in size or physical property change is not large.

In one embodiment, the first material may be a material that shows high light transmittance from an ultraviolet region to a visible light region and a near infrared region, and a maximum intensity of autofluorescence when excited light is irradiated is smaller than the standard deviation of background noise when excited light is not irradiated.

In one embodiment, the plurality of tubes may include the second tube corresponding to the distal tip 130 region from which light is emitted to the internal body tissue among the plurality of tubes of the imaging sheath. The first tube and the second tube may constitute the imaging sheath. For example, the imaging sheath may be formed in a manner in which the first tube and the second tube are coupled.

In one embodiment, the second tube may be made of a second material in which a maximum intensity of autofluorescence when excited light is irradiated is larger than the standard deviation of background noise when excited light is not irradiated. For example, the second material may be a copolymer composed of two blocks of Polyether and Polyamide. for example, the second material may be a copolymer called Pebax. For example, the second material may correspond to a product having a trademark name of Pebax as a type of high-performance thermoplastic elastomer.

In one embodiment, the second material exhibits autofluorescence characteristics when light of a specific wavelength (for example, a wavelength in an ultraviolet region) is irradiated, and the autofluorescence of the second material may act as an unwanted signal (noise) generated by the second material itself during the imaging operation of the fluorescence lifetime imaging catheter 100.

In the present disclosure, specific methods for reducing autofluorescence generated by the sheath itself are described with reference to the following figures.

In one embodiment, when the fluorescence lifetime imaging catheter 100 is inserted into the body, the first tube in the imaging sheath is located proximal to the internal body tissue, and the second tube may be located distal to the internal body tissue.

In one embodiment, the fluorescence lifetime imaging catheter 100 may be connected to a device including a processor (not shown) and a memory (not shown) to perform a fluorescence lifetime imaging operation.

In one embodiment, the processor may process an overall operation related to fluorescence lifetime imaging, such as analyzing a fluorescence lifetime from an image acquired from the fluorescence lifetime imaging catheter 100. The processor may acquire signals such as autofluorescence, light, and a wavelength through the constituent element (e.g., the focusing device 150) examined above, and may provide or process functions such as generating data to be provided to a user using the acquired signals.

In one embodiment, the memory may include a storage medium of at least one type among a flash memory type, a hard disk type, a multimedia card micro type, a card type memory (e.g., SD or XD memory, etc.), random access memory (RAM), static random access memory (SRAM), read-only memory (ROM), electrically erasable programmable read-only memory (EEPROM), programmable read-only memory (PROM), a magnetic memory, a magnetic disk, and an optical disc.

In one embodiment, the listed constituent elements are not essential in implementing the fluorescence lifetime imaging catheter 100, so the fluorescence lifetime imaging catheter 100 may have more or less constituent elements than the constituent elements listed above.

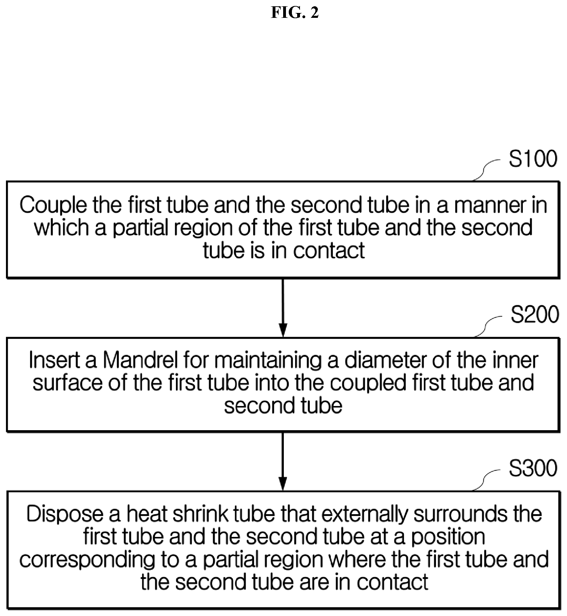

FIG. 2 is a flowchart for explaining a method for coupling some configurations of the fluorescence lifetime imaging catheter according to some embodiments of the present disclosure.

In the step of FIG. 2, for example, a method for coupling the first tube and the second tube constituting the imaging sheath of the fluorescence lifetime imaging catheter is exemplarily illustrated.

In S100, the first tube and the second tube may be coupled in a manner in which at least a part of an outer surface of the second tube is inserted into an inner surface of the first tube such that a partial region of the first tube and the second tube is in contact.

In one embodiment, in order for the first tube and the second tube to be coupled, a diameter of the inner surface of the first tube is expanded, and an outer surface of the second tube may be inserted into an expanded inner diameter of the first tube.

In S200, a Mandrel for maintaining a diameter of the inner surface of the first tube may be inserted into the coupled first tube and second tube during a coupling process of the first tube and the second tube.

In one embodiment, the Mandrel may be a cylindrical material made of a material resistant to high temperature heat. For example, a diameter of the Mandrel may be equal to or smaller than the diameter of the inner surface of the first tube. By inserting the Mandrel into the inner surface of the coupled (e.g., contacted or attached) first tube and second tube, a coupling process between the first tube and the second tube may be performed while the coupling shape between the first tube and the second tube is maintained.

In S300, a heat shrink tube that externally surrounds the first tube and the second tube may be disposed at a position corresponding to a partial region where the first tube and the second tube are in contact during the coupling process of the first tube and the second tube. By inserting the heat shrink tube into the outer surface of the coupled (e.g., contacted or attached) first tube and second tube (that is, by inserting an assembly of the first tube and the second tube into an inner surface of the heat shrink tube), a heating treatment process and a cooling treatment process may be applied to the outer surface of the assembly of the first tube and the second tube through the heat shrink tube.

In one embodiment, the heat shrink tube may be a material whose physical shape is changed by hot air and/or cold air.

In one embodiment, by changing the physical shape of the heat shrink tube, the first tube and the second tube may be coupled such that the coupling between the first tube and the second tube can withstand a force (e.g., 3N) equal to or greater than a specific reference size.

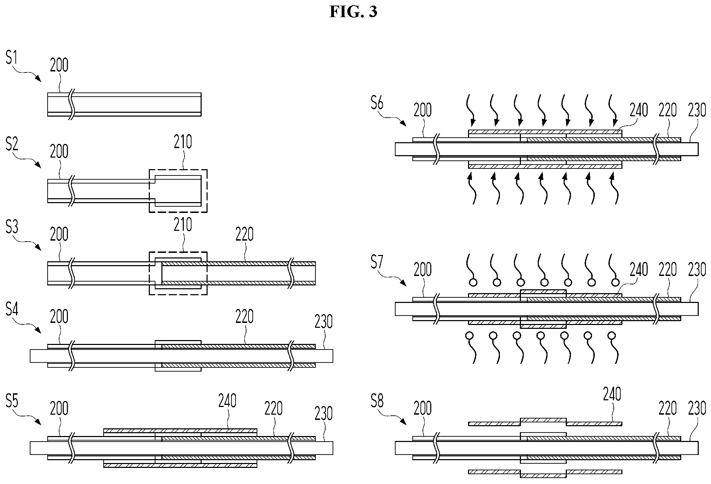

FIG. 3 is a diagram for explaining a method for coupling some configurations of the fluorescence lifetime imaging catheter according to some embodiments of the present disclosure.

An imaging sheath of the fluorescence lifetime imaging catheter according to one embodiment of the present disclosure may be configured by bonding different tubes having several characteristics. Accordingly, noise due to autofluorescence generated in the imaging sheath itself during an imaging operation of the imaging catheter may be removed.

When coupling different tubes (e.g., bonding them), a bonding method of applying a heat shrink tube after flaring an EFEP tube (e.g., a first tube) to widen an inner diameter and then inserting another tube (e.g., a second tube) to be bonded may be used in the present disclosure to increase bonding strength. In this case, a Mandrel having an appropriate diameter (e.g., a diameter corresponding to the inner diameter of the second tube) is used to prevent the inner diameter of the first tube and the second tube from being reduced during bonding, and after the bonding, the heat shrink tube is removed, whereby an assembly of the first tube and the second tube with enhanced bonding strength may be generated.

In S1, the first tube (200) may be provided by being cut to an appropriate length.

In S2, heat is applied to one end (210) of the cut first tube (200) for an appropriate length (flaring it), whereby an inner diameter of the inner surface may be expanded.

In S3, the first tube (200) and the second tube (220) may be coupled by inserting a part of the second tube (220) into the end (210) of the expanded first tube (200).

In S4, a Mandrel (230) may be inserted into the coupled first tube (200) and the second tube (220).

In S5, a heat shrink tube (240) may be worn externally in a state where the Mandrel (230) is inserted into the first tube (200) and the second tube (220).

In S6, in a state where the heat shrink tube (240) is worn, the heat shrink tube (240), which is softened by applying hot air of a specific temperature range, may be contracted by reducing the diameter. The bonding force of the first tube (200) and the second tube (220) may be reinforced by the contracted heat shrink tube (240).

In S7, the heat shrink tube (240) may be cooled down and hardened by applying cold air to the heat shrink tube (240) whose temperature has risen due to the hot air. As the heat shrink tube (240) is cooled down and hardened, the assembly of the first tube (200) and the second tube (220) may also be cooled down, and a shape of the assembly may be hardened.

In S8, the hardened heat shrink tube (240) may be separated/removed, and the coupling of the first tube (200) and the second tube (220) may be completed.

In one embodiment, the first tube (200) and/or the second tube (220) may be a single tube or a tube composed of multiple layers.

The imaging catheter may be manufactured in a manner exemplified in FIG. 3. Accordingly, a technical effect may be achieved in that autofluorescence generated by the imaging sheath of the catheter is removed, and a coupling force of the tubes in the imaging sheath may also be reinforced.

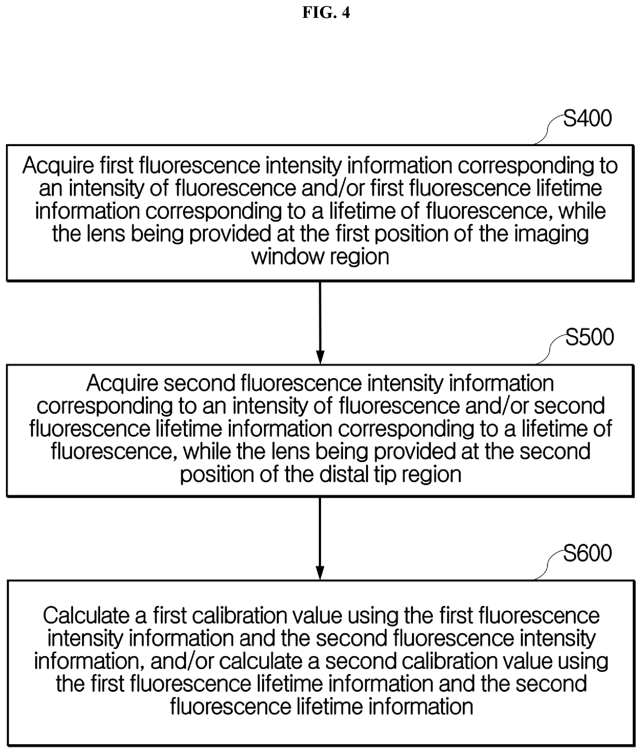

FIG. 4 is a flowchart for explaining a method for calculating a calibration value using the fluorescence lifetime imaging catheter according to some embodiments of the present disclosure.

In the fluorescence lifetime imaging technology, since the acquired fluorescence intensity or fluorescence lifetime may vary depending on a length of an optical fiber inside the catheter and a quality of a reflective surface, calibration needs to be performed so that the same fluorescence intensity and fluorescence lifetime can be acquired and calculated even if the catheter changes for the same lesion. To this end, a standard specimen whose fluorescence intensity or fluorescence lifetime is known in advance must be photographed under the same conditions, but a problem may arise in that time taken to photograph the separate standard specimen may be burdensome and inconvenient when a quick procedure is required. In the present disclosure, a methodology for calculating a calibration value using the fluorescence intensity and fluorescence lifetime of the tube is presented by applying a tube having autofluorescence characteristics (e.g., a Pebax material second tube) to a distal tip region of the imaging sheath instead of the standard specimen whose fluorescence intensity or fluorescence lifetime is known in advance. For example, the second tube having fluorescence characteristics may be a tube coated with a fluorescent substance or may be a tube made of a material having autofluorescence characteristics without applying a separate fluorescent substance.

In one embodiment, during a calibration operation, the focusing device (150) of the imaging core is located inside the distal tip region (e.g., a Pebax material tube), and the tube of the distal tip region has fluorescence characteristics, so fluorescence intensity or fluorescence lifetime information of the distal tip region can be acquired through the focusing device (150). Also, during imaging, the focusing device (150) in the imaging core is positioned in the tube of the imaging window region (e.g., the first tube), so fluorescence information of the tubular body tissue can be acquired without interference of autofluorescence of the imaging sheath.

In one embodiment, a calibration value may be calculated using a value when the focusing device (150) is positioned in the second tube and a value when the focusing device (150) is positioned in the first tube.

In one embodiment, since it is physically impossible for the internal optical fiber length to be perfectly the same for each catheter, a technique according to one embodiment of the present disclosure may solve a problem that the lengths of the catheters may be partially different by calibrating it, and a technical effect that accuracy of an imaging result can be guaranteed may be achieved. The present disclosure performs an imaging operation in a manner of compensating for a difference between an ideal fluorescence lifetime and an actually acquired fluorescence lifetime by photographing a specimen whose ideal fluorescence lifetime information is known in advance during a calibration process, so a more accurate imaging result can be acquired.

Referring to FIG. 4, the fluorescence lifetime imaging catheter (100) may compensate for noise due to autofluorescence generated by the imaging sheath itself (e.g., the second tube) during the imaging operation of the fluorescence lifetime imaging catheter (100) for the internal body tissue using a predefined calibration value. This calibration value may be used to guarantee accuracy of the imaging operation of the fluorescence lifetime imaging catheter (100). To calculate the calibration value, the following steps in FIG. 4 may be performed. The steps of FIG. 4 may be performed by the fluorescence lifetime imaging catheter (100).

In one embodiment, the fluorescence lifetime imaging catheter (100) may move a position of the focusing device (150) in the fluorescence lifetime imaging catheter (100) using an external driving unit or driving mechanism. For example, the focusing device (150) may be moved between the imaging window region (120) of the imaging sheath and the distal tip region (130) of the imaging sheath.

In one embodiment, the position of the focusing device (150) may be variably determined according to a type of the operation of the fluorescence lifetime imaging catheter (100). For example, when the operation of the fluorescence lifetime imaging catheter (100) is a calibration operation that calculates a calibration value, the focusing device (150) may be moved between the position corresponding to the imaging window region (120) and the position corresponding to the distal tip (130). Accordingly, a technical effect may be achieved in that autofluorescence of the imaging sheath can be efficiently used during the calibration operation. For example, when the operation of the fluorescence lifetime imaging catheter (100) is an imaging operation for the body tissue, the focusing device (150) may be positioned at the position corresponding to the imaging window region (120). Accordingly, a technical effect may be achieved in that autofluorescence of the imaging sheath can be efficiently removed (e.g., noise generated by the sheath itself can be effectively removed) during the imaging operation. Also, during the calibration operation of the fluorescence lifetime imaging catheter (100), a technical effect may be achieved in that a calibration value can be effectively acquired using the fluorescence intensity and fluorescence lifetime of the distal tip (130) acquired in advance.

In one embodiment, to acquire the calibration value, in S400, the fluorescence lifetime imaging catheter (100) may acquire first fluorescence intensity information corresponding to an intensity of fluorescence and/or first fluorescence lifetime information corresponding to a lifetime of fluorescence in a state where the focusing device (150) is provided at the first position of the imaging window region (120).

In one embodiment, to acquire the calibration value, in S500, the fluorescence lifetime imaging catheter (100) may acquire second fluorescence intensity information corresponding to an intensity of fluorescence and/or second fluorescence lifetime information corresponding to a lifetime of fluorescence in a state where the focusing device (150) is provided at the second position of the distal tip (130) region.

In one embodiment, to acquire the calibration value, in S600, the fluorescence lifetime imaging catheter (100) may calculate a first calibration value using the first fluorescence intensity information and the second fluorescence intensity information, and/or may calculate a second calibration value using the first fluorescence lifetime information and the second fluorescence lifetime information. The fluorescence lifetime imaging catheter (100) may calculate the first calibration value using the first fluorescence intensity information and the second fluorescence intensity information as parameters of a calibration function, and/or may calculate the second calibration value using the first fluorescence lifetime information and the second fluorescence lifetime information as parameters of the calibration function. Accordingly, a technical effect may be achieved in that accuracy of a fluorescence lifetime imaging result can be guaranteed as the first calibration value related to fluorescence intensity and the second calibration value related to fluorescence lifetime are applied to the fluorescence lifetime imaging result.

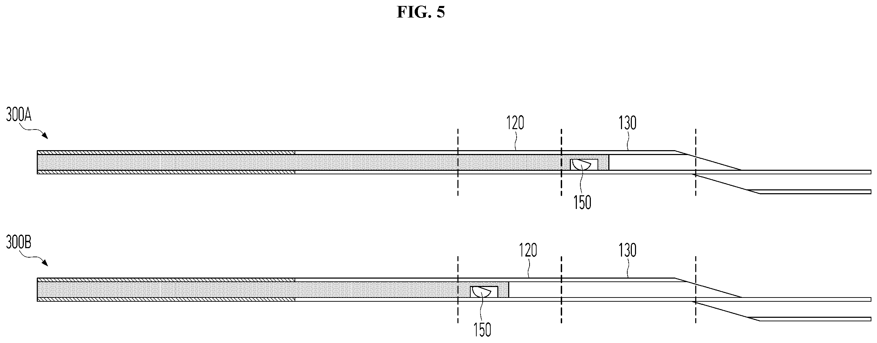

FIG. 5 is a diagram for explaining a method for calculating a calibration value using a fluorescence lifetime imaging catheter according to some embodiments of the present disclosure.

Referring to FIG. 5, the fluorescence lifetime imaging catheter (100) may move the focusing device (150) of the imaging core from a first position in an imaging window area (120) corresponding to a first tube, at 300B, to a second position in the distal tip (130) area corresponding to a second tube, at 300A, in order to calculate the calibration value. For example, the fluorescence lifetime imaging catheter (100) may move the focusing device (150) using an external piezo actuator, solenoid, and/or motor, or may move the focusing device (150) using a fluid-driven method, a metal thermal expansion method, a method using electrostatic force, and/or a method using magnetic force.

In one embodiment, the calibration value may include a first calibration value (c) calculated based on first fluorescence intensity information (Im) corresponding to the intensity of fluorescence obtained when the focusing device (150) is located at the first position of the imaging window area (120), and second fluorescence intensity information (Ir) corresponding to the intensity of fluorescence obtained when the focusing device (150) is located at the second position of the distal tip (130) area.

In one embodiment, the fluorescence lifetime imaging catheter (100) can consistently perform the fluorescence intensity measurement of the target tissue independent of the catheter's performance by multiplying the first calibration value by the obtained fluorescence intensity of the target tissue. In an additional embodiment, the fluorescence lifetime imaging catheter (100) can consistently perform the fluorescence intensity measurement of the target tissue independent of the catheter's performance by subtracting the first calibration value from the obtained fluorescence intensity of the target tissue.

In one embodiment, the calibration value may include a second calibration value (tc) calculated based on first fluorescence lifetime information (τm) corresponding to the lifetime of fluorescence obtained when the focusing device (150) is located at the first position of the imaging window area (120), and second fluorescence lifetime information (τr) corresponding to the lifetime of fluorescence obtained when the focusing device (150) is located at the second position of the distal tip (130) area.

In one embodiment, the fluorescence lifetime imaging catheter (100) can perform correction for the fluorescence lifetime of the target tissue by adding the second calibration value to the obtained fluorescence lifetime of the target tissue. In an additional embodiment, the fluorescence lifetime imaging catheter (100) can consistently perform the fluorescence lifetime measurement of the target tissue independent of the catheter's performance by subtracting the second calibration value from the obtained fluorescence lifetime of the target tissue.

In one embodiment, the fluorescence lifetime imaging catheter (100) can consistently perform the fluorescence lifetime measurement of the target tissue independent of the catheter's performance by applying the calibration value to the fluorescence information obtained when the focusing device (150) is located at the first position during the imaging operation of the fluorescence lifetime imaging catheter (100) on internal body tissue.

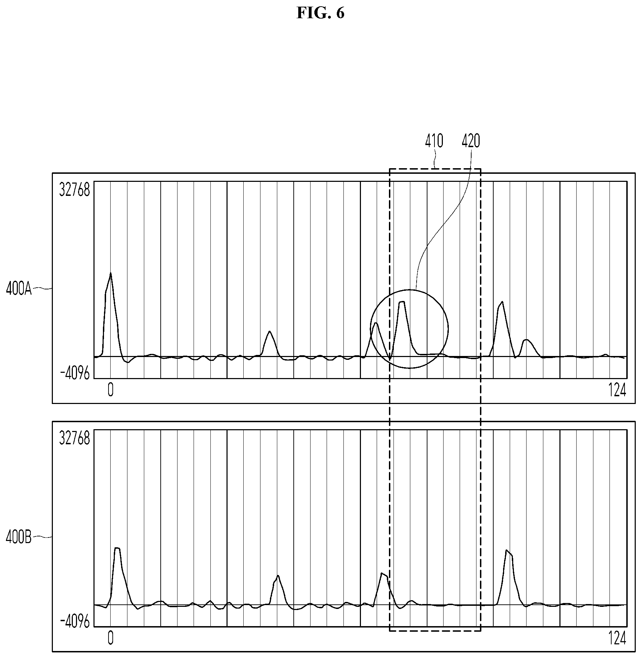

FIG. 6 is a diagram exemplarily illustrating a result when imaging is performed using a fluorescence lifetime imaging catheter according to some embodiments of the present disclosure.

Referring to FIG. 6, the result (400A), which indicates the fluorescence intensity-related value when the first material in the present disclosure is not applied to the first tube, and the result (400B), which indicates the fluorescence intensity-related value when the first material in the present disclosure is applied to the first tube, are comparably displayed.

As shown in FIG. 6, it can be confirmed that noise corresponding to reference number 420 in the area 410 occurs in result (400A). This noise may correspond to noise due to autofluorescence generated by the imaging sheath itself. It can be confirmed that no noise is generated in the area 410 in result (400B). This may result from the configuration of the first tube, corresponding to the imaging window area of the imaging sheath, with the first material.

In one embodiment, referring to FIG. 6, a time (x-axis) versus fluorescence intensity (y-axis) graph (400A) obtained when the imaging window area (120) is the second material, and a time (x-axis) versus fluorescence intensity (y-axis) graph (400B) obtained when the imaging window area (120) is the first material are disclosed.

In one embodiment, the target signal intended to be acquired through the graph (400) is the area corresponding to the dashed line (410), and when the imaging window area (120) is the second material, noise (420) corresponding to an unwanted signal acquired due to the second material may be included.

In one embodiment, when the imaging window area (120) is the first material, it exhibits high light transmittance from the ultraviolet region to the visible light and near-infrared regions and does not show autofluorescence characteristics; thus, noise (not shown) may not be acquired in graph (400B).

The first tube formed with the first material, the attachment method of the first tube formed with the first material, and the calibration using the first material proposed in the present disclosure to remove/cancel noise generated by the fluorescence lifetime imaging catheter (100) itself can be applied to all non-limiting optical imaging catheters for imaging tubular body tissue.

In one embodiment, the first material may exhibit high light transmittance over a wavelength range corresponding to the ultraviolet-visible light-near-infrared regions. Accordingly, the first tube configured with the first material does not affect the IV-OCT signal, has no wavelength constraint, and can be applied to any multimodal OCT, thereby producing the effect of being applicable to any multimodal OCT.

In one embodiment, by using the first tube formed with the first material, the wavelength acquired by the fluorescence lifetime imaging catheter (100) may have a low noise level despite high transmittance. That is to say, since high light efficiency and an excellent signal-to-noise ratio (SNR) can be achieved through the first tube formed with the first material, higher-quality images can be provided to the user, thereby producing the effect of increasing diagnostic accuracy and sensitivity.

Furthermore, the method of attaching the first tube can increase the success rate of the procedure by allowing the user to perform the procedure with confidence, thereby resulting in the effect of increasing patient safety.

In addition, by calculating the calibration value and applying it to the obtained fluorescence signal, image differences due to catheter differences are reduced, and images with higher reproducibility can be obtained, thereby producing the effect of increasing diagnostic accuracy.

The catheter according to one embodiment of the present disclosure is a method for reducing artifacts caused by an autofluorescence source (e.g., an imaging sheath), and by blocking the source of noise to be reduced, the technical effect of implementing a more accurate fluorescence lifetime imaging operation can be achieved.

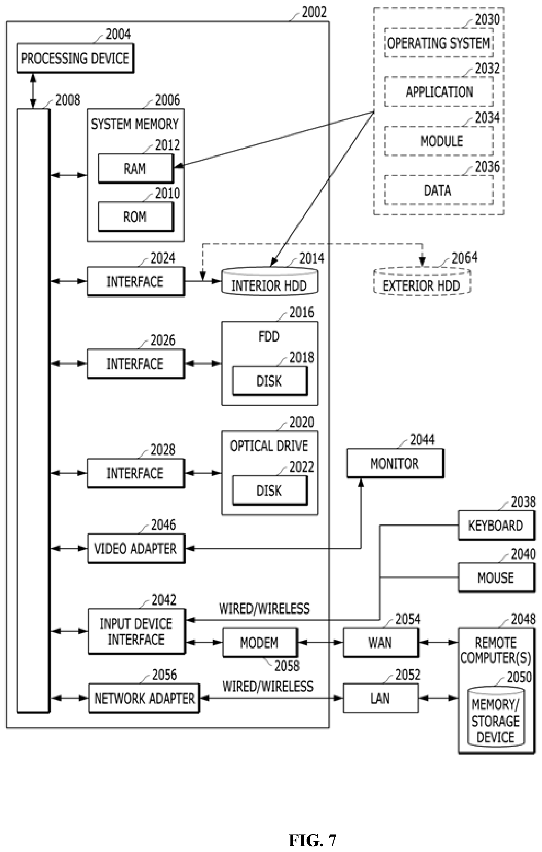

FIG. 7 illustrates a general schematic diagram for an exemplary computing environment in which embodiments of the present disclosure may be implemented.

The computing device in FIG. 7 may correspond to a computing device that is connectable or communicable with the imaging catheter (100). One skilled in the art will appreciate that the computing device of the present disclosure may be implemented in combination with computer-executable instructions and/or other program modules that can be executed on one or more computers and/or as a combination of hardware and software. Generally, a program module includes routines, programs, components, data structures, and the like that perform specific tasks or implement specific abstract data types. Furthermore, one skilled in the art will appreciate that the method of the present disclosure may be implemented in other computer system configurations, including single-processor or multiprocessor computer systems, minicomputers, mainframe computers, as well as personal computers, handheld (handheld) computing devices, microprocessor-based or programmable consumer electronics, and the like (each of which may operate in connection with one or more associated devices).

The described embodiments of the present disclosure may also be implemented in a distributed computing environment where certain tasks are performed by remote processing devices connected via a communication network. In a distributed computing environment, program modules can be located in both local and remote memory storage devices.

The computing device, computing apparatus, computer, system, component, module, or unit in the present disclosure includes routines, procedures, programs, components, data structures, and the like that perform a specific task or implement a specific abstract data type. Furthermore, one skilled in the art will fully recognize that the methods presented in the present disclosure may be implemented in other computer system configurations, including single-processor or multiprocessor computing devices, minicomputers, mainframe computers, as well as personal computers, handheld computing devices, microprocessor-based or programmable consumer electronics, and the like (each of which may operate in connection with one or more associated devices).

The described embodiments in the present disclosure may also be implemented in a distributed computing environment where certain tasks are performed by remote processing devices connected via a communication network. In a distributed computing environment, program modules can be located in both local and remote memory storage devices.

Computing devices typically include various computer-readable media. Any medium that is accessible by a computer can be a computer-readable medium, and such computer-readable media includes volatile and non-volatile media, transitory and non-transitory media, and removable and non-removable media. By way of example, and not limitation, computer-readable media may comprise computer-readable storage media and computer-readable transmission media.

Computer-readable storage media includes volatile and non-volatile media, temporary and non-transitory media, and removable and non-removable media implemented in any method or technology for storing information, such as computer-readable instructions, data structures, program modules, or other data. Computer-readable storage media includes, but is not limited to, RAM, ROM, EEPROM, flash memory or other memory technology, CD-ROM, DVD (digital video disk) or other optical disk storage devices, magnetic cassettes, magnetic tape, magnetic disk storage devices or other magnetic storage devices, or any other medium that can be accessed by a computer and used to store the desired information.

Computer-readable transmission media typically embodies computer-readable instructions, data structures, program modules, or other data in a modulated data signal, such as a carrier wave or other transport mechanism, and includes all information delivery media. The term "modulated data signal" means a signal that has one or more of its characteristics set or changed in such a manner as to encode information in the signal. By way of example, and not limitation, computer-readable transmission media includes wired media such as a wired network or a direct-wired connection, and wireless media such as acoustic, RF, infrared, and other wireless media. Combinations of any of the above should also be included within the scope of computer-readable transmission media.

An exemplary environment (2000) for implementing various aspects of the present invention, including a computer (2002), is shown, and the computer (2002) includes a processing unit (2004), system memory (2006), and a system bus (2008). The computer (200) in the present specification may be used interchangeably with the computing device. The system bus (2008) connects system components including, system memory (2006) (not limited to) to the processing unit (2004). The processing unit (2004) can be any of a variety of commercially available processors. Dual processors and other multiprocessor architectures can also be used as the processing unit (2004).

The system bus (2008) can be any of several types of bus structures that may be further interconnected to a memory bus, a peripheral bus, and a local bus using any of a variety of commercially available bus architectures. System memory (2006) includes read only memory (ROM) (2010) and random access memory (RAM) (2012). A basic input/output system (BIOS) is stored in a nonvolatile memory (2010) such as ROM, EPROM, EEPROM, and the like, and the BIOS contains basic routines that help transfer information between components within the computer (2002), such as during startup. RAM (2012) may also include high speed RAM such as static RAM for caching data.

The computer (2002) also includes an internal hard disk drive (HDD) (2014) (e.g., EIDE, SATA), an external hard disk (e.g., USB, Thunderbolt, eSATA) (2064), a magnetic floppy disk drive (FDD) (2016) (e.g., for reading from or writing to a removable diskette (2018)), an SSD, and an optical disk drive (2020) (e.g., for reading a CD-ROM disk (2022) or for reading from or writing to other high-capacity optical media such as a DVD). The hard disk drives (2014 and 2064), magnetic disk drive (2016), and optical disk drive (2020) are each connected to the system bus (2008) by a hard disk drive interface (2024), a magnetic disk drive interface (2026), and an optical drive interface (2028), respectively. The interface (2024) for external drive implementation includes, for example, at least one or both of USB (Universal Serial Bus) and IEEE 1394 interface technologies.

These drives and their associated computer-readable media provide nonvolatile storage of data, data structures, computer-executable instructions, and the like. For the computer (2002), the drives and media correspond to storing any data in a suitable digital format. Although the description of computer-readable storage media above refers to HDD, removable magnetic disk, and removable optical media such as CD or DVD, one skilled in the art will appreciate that other types of computer-readable storage media such as a zip drive, magnetic cassette, flash memory card, cartridge, and the like can also be used in the exemplary operating environment, and that any such media can contain computer-executable instructions for performing the methods of the present invention.

A number of program modules, including an operating system (2030), one or more application programs (2032), other program modules (2034), and program data (2036) can be stored on the drives and RAM (2012). All or a portion of the operating system, applications, modules, and/or data may also be cached in RAM (2012). It will be appreciated that the present invention can be implemented in a variety of commercially available operating systems or combinations of operating systems.

A user can enter commands and information into the computer (2002) through one or more wired/wireless input devices, for example, a pointing device such as a keyboard (2038) and a mouse (2040). Other input devices (not shown) may include a microphone, an IR remote control, a joystick, a game pad, a stylus pen, a touch screen, and the like. These and other input devices are often connected to the processing unit (2004) through an input device interface (2042) connected to the system bus (2008), but may be connected by other interfaces such as a parallel port, an IEEE 1394 serial port, a game port, a USB port, an IR interface, and the like.

A monitor (2044) or other type of display device is also connected to the system bus (2008) via an interface such as a video adapter (2046). In addition to the monitor (2044), the computer typically includes other peripheral output devices (not shown) such as speakers, a printer, and the like.

The computer (2002) can operate in a networked environment using logical connections to one or more remote computers (2048), such as remote computer(s) (2048), via wired and/or wireless communication. The remote computer(s) (2048) may be a workstation, a server computer, a router, a personal computer, a portable computer, a microprocessor-based entertainment appliance, a peer device, or other common network node, and generally includes many or all of the components described with respect to the computer (2002), but for brevity, only a memory storage device (2050) is illustrated. The logical connections illustrated include a wired/wireless connection to a local area network (LAN) (2052) and/or a larger network, for example, a wide area network (WAN) (2054). Such LAN and WAN networking environments are common in offices and companies, facilitate an enterprise-wide computer network such as an intranet, all of which may be connected to a worldwide computer network, for example, the Internet.

When used in a LAN networking environment, the computer (2002) is connected to a local network (2052) through a wired and/or wireless communication network interface or adapter (2056). The adapter (2056) can facilitate wired or wireless communication to the LAN (2052), which also includes a wireless access point installed therein for communicating with the wireless adapter (2056). When used in a WAN networking environment, the computer (2002) may include a modem (2058), be connected to a communication server on the WAN (2054), or have other means for establishing communication through the WAN (2054), such as over the Internet. The modem (2058), which can be an internal or external and wired or wireless device, is connected to the system bus (2008) via a serial port interface (2042). In a networked environment, program modules described with respect to the computer (2002) or portions thereof may be stored in remote memory/storage device (2050). It will be appreciated that the network connections shown are exemplary and other means of establishing a communication link between the computers can be used.

The computer (1602) operates to communicate with any wireless device or entity deployed in wireless communication, for example, a printer, a scanner, a desktop and/or portable computer, a PDA (portable data assistant), a communication satellite, any equipment or location associated with a wireless detectable tag, and a telephone. This includes at least Wi-Fi and Bluetooth wireless technologies. Therefore, communication can be a predefined structure, as in a conventional network, or simply an ad hoc communication between at least two devices.

Wi-Fi (Wireless Fidelity) allows connection to the Internet and the like without wires. Wi-Fi is a wireless technology, such as a cell phone, that allows such a device, for example, a computer, to transmit and receive data anywhere indoors and outdoors, that is, within the service area of a base station. A Wi-Fi network uses a wireless technology called IEEE 802.11 (a, b, g, and the like) to provide a secure, reliable, and high-speed wireless connection. Wi-Fi can be used to connect computers to each other, to the Internet, and to wired networks (using IEEE 802.3 or Ethernet). Wi-Fi networks can operate at unauthorized 2.4 and 5GHz wireless bands, for example, at 11Mbps (802.11a) or 54 Mbps (802.11b) data rates, or can operate in products including both bands (dual-band).

One skilled in the art of the present disclosure will understand that information and signals can be represented using any of a variety of different technologies and techniques. For example, data, instructions, commands, information, signals, values, symbols, and chips that may be referred to in the above description may be represented by voltages, currents, electromagnetic waves, magnetic fields or particles, optical fields or particles, or any combination thereof.

One skilled in the art of the present disclosure will understand that various exemplary logical blocks, modules, processors, means, circuits, and algorithm steps described in connection with the embodiments disclosed herein may be implemented by electronic hardware, program or design code in various forms (referred to herein as software for convenience), or a combination of both. To clearly explain this interchangeability of hardware and software, various exemplary components, blocks, modules, circuits, and steps have been described above generally in terms of their functionality. Whether such functionality is implemented as hardware or software depends upon the particular application and design constraints imposed on the overall system. One skilled in the art of the present disclosure can implement the described functionality in a variety of ways for each particular application, but such implementation decisions should not be interpreted as departing from the scope of the present disclosure.

The various embodiments presented herein may be implemented as a method, apparatus, or article of manufacture using standard programming and/or engineering techniques. The term article of manufacture includes a computer program, carrier, or media accessible from any computer-readable storage device. For example, the computer-readable storage media includes, but is not limited to, magnetic storage devices (e.g., hard disk, floppy disk, magnetic strip, etc.), optical disks (e.g., CD, DVD, etc.), smart cards, and flash memory devices (e.g., EEPROM, card, stick, key drive, etc.). Furthermore, the various storage media presented herein include one or more devices and/or other machine-readable media for storing information.

It is to be understood that the specific order or hierarchy of steps in the processes presented is an example of exemplary approaches. It is to be understood that based on design priorities, the specific order or hierarchy of steps in the processes may be rearranged within the scope of the present disclosure. The accompanying method claims provide elements of various steps in a sample order but are not intended to be limited to the specific order or hierarchy presented.

The description of the presented embodiments is provided to enable any person skilled in the art to use or implement the present disclosure. Various modifications to these embodiments will be apparent to those skilled in the art of the present disclosure, and the general principles defined herein may be applied to other embodiments without departing from the scope of the present disclosure. Thus, the present disclosure is not intended to be limited to the embodiments presented herein, but is to be interpreted in the widest scope consistent with the principles and novel features presented herein.

The description of the presented embodiments is provided to enable any person skilled in the art to use or implement the present disclosure. Various modifications to these embodiments will be apparent to those skilled in the art of the present disclosure, and the general principles defined herein may be applied to other embodiments without departing from the scope of the present disclosure. Thus, the present disclosure is not intended to be limited to the embodiments presented herein, but is to be interpreted in the widest scope consistent with the principles and novel features presented herein.

## Claims

1. A multimodal imaging catheter inserted into a body to acquire information of an internal body tissue, comprising:
an imaging core including a double clad fiber and a focusing device, wherein the imaging core transfers light to the internal body tissue and collects information within the internal body tissue; and
an imaging sheath including the imaging core therein to protect the imaging core from an external environment and to separate the internal body tissue and the imaging core during an imaging operation of the multimodal imaging catheter for the internal body tissue, and
wherein the imaging sheath includes a plurality of tubes coupled along a longitudinal direction of the multimodal imaging catheter,
a first tube corresponding to an imaging window region through which light generated by a rotation of the focusing device passes among a plurality of tubes of the imaging sheath is made of a first material in which a maximum intensity of autofluorescence when excited light is irradiated, is smaller than a standard deviation of background noise when the excited light is not irradiated, in order to reduce an autofluorescence generated by the imaging sheath itself,
a second tube corresponding to a distal tip region from which the light is emitted to the internal body tissue among a plurality of tubes of the imaging sheath is made of a second material in which a maximum intensity of autofluorescence when excited light is irradiated, is larger than a standard deviation of background noise when excited light is not irradiated, and
when the multimodal imaging catheter is inserted into a body, the first tube in the imaging sheath is located proximal to the internal body tissue, and the second tube is located distal to the internal body tissue.

2. The multimodal imaging catheter of claim 1, wherein the first material is enhanced fluorinated ethylene propylene.

3. The multimodal imaging catheter of claim 2, wherein the second material is a copolymer composed of two blocks of polyether and polyamide.

4. The multimodal imaging catheter of claim 1, wherein the first tube and the second tube are coupled in a manner in which at least a part of an outer surface of the second tube is inserted into an inner surface of the first tube such that a partial region of the first tube and the second tube is in contact, and a mandrel for maintaining a diameter of an inner surface of the first tube is inserted into the coupled first tube and second tube during a coupling process of the first tube and the second tube.

5. The multimodal imaging catheter of claim 4, wherein a heat shrink tube that externally surrounds the first tube and the second tube is disposed at a position corresponding to the partial region where the first tube and the second tube are in contact during a coupling process of the first tube and the second tube, and
the heat shrink tube couples the first tube and the second tube such that a coupling between the first tube and the second tube can withstand a force equal to or greater than a reference size by physically changing its shape by hot air or cold air.

6. The multimodal imaging catheter of claim 1, wherein the focusing device of the imaging core is positioned in the imaging window region corresponding to the first tube during the imaging operation of the multimodal imaging catheter for the internal body tissue.

7. The multimodal imaging catheter of claim 1, wherein the multimodal imaging catheter calculates a calibration value of the multimodal imaging catheter using a predefined fluorescence lifetime value of an autofluorescence of the second tube before the imaging operation for the internal body tissue.

8. The multimodal imaging catheter of claim 7, wherein the focusing device of the imaging core is moved from a first position in the imaging window region corresponding to the first tube to a second position in the distal tip region corresponding to the second tube to calculate the calibration value of the multimodal imaging catheter.

9. The multimodal imaging catheter of claim 7, wherein the calibration value includes a first calibration value calculated based on first fluorescence intensity information corresponding to an intensity of fluorescence acquired while the focusing device is provided at a first position of the imaging window region and second fluorescence intensity information corresponding to an intensity of fluorescence acquired while the focusing device is provided at a second position of the distal tip region.

10. The multimodal imaging catheter of claim 7, wherein the calibration value includes a second calibration value calculated based on first fluorescence lifetime information corresponding to a lifetime of fluorescence acquired while the focusing device is provided at a first position of the imaging window region and second fluorescence lifetime information corresponding to a lifetime of fluorescence acquired while the focusing device is provided at a second position of the distal tip region.

11. The multimodal imaging catheter of claim 9 or claim 10, wherein the multimodal imaging catheter calibrates fluorescence intensity and fluorescence lifetime acquired by the multimodal imaging catheter by applying the calibration value to fluorescence information acquired while the focusing device is provided at the first position during the imaging operation of the multimodal imaging catheter for the internal body tissue.

12. A fluorescence lifetime imaging catheter inserted into a body to measure a fluorescence lifetime of an internal body tissue, comprising:
an imaging core including an optical fiber and a focusing device, wherein the imaging core transfers light to the internal body tissue and collects fluorescence emitted by a fluorescent substance within the internal body tissue; and
an imaging sheath including the imaging core therein to protect the imaging core from an external environment and to separate the internal body tissue and the imaging core during an imaging operation of the fluorescence lifetime imaging catheter for the internal body tissue, and
wherein the imaging sheath includes a plurality of tubes coupled along a longitudinal direction of the fluorescence lifetime imaging catheter,
a first tube corresponding to an imaging window region through which light generated by a rotation of the focusing device passes among a plurality of tubes of the imaging sheath, is made of a first material in which a maximum intensity of autofluorescence when excited light is irradiated, is smaller than a standard deviation of background noise when excited light is not irradiated, in order to reduce an autofluorescence generated by the imaging sheath itself,
a second tube corresponding to a distal tip region from which the light is emitted to the internal body tissue among a plurality of tubes of the imaging sheath, is made of a second material in which a maximum intensity of autofluorescence when excited light is irradiated, is larger than a standard deviation of background noise when excited light is not irradiated, and
when the fluorescence lifetime imaging catheter is inserted into a body, the first tube in the imaging sheath is located proximal to the internal body tissue, and the second tube is located distal to the internal body tissue.

13. The fluorescence lifetime imaging catheter of claim 12, wherein the first material is enhanced fluorinated ethylene propylene.

14. The fluorescence lifetime imaging catheter of claim 13, wherein the second material is a copolymer composed of two blocks of polyether and polyamide.

15. The fluorescence lifetime imaging catheter of claim 12, wherein the first tube and the second tube are coupled in a manner in which at least a part of an outer surface of the second tube is inserted into an inner surface of the first tube such that a partial region of the first tube and the second tube is in contact, and a mandrel for maintaining a diameter of the inner surface of the first tube is inserted into the coupled first tube and second tube during a coupling process of the first tube and the second tube.

16. The fluorescence lifetime imaging catheter of claim 15, wherein a heat shrink tube that externally surrounds the first tube and the second tube is disposed at a position corresponding to the partial region where the first tube and the second tube are in contact during a coupling process of the first tube and the second tube, and
the heat shrink tube couples the first tube and the second tube such that a coupling between the first tube and the second tube can withstand a force equal to or greater than a reference size by physically changing its shape by hot air or cold air.

17. The fluorescence lifetime imaging catheter of claim 12, wherein the focusing device of the imaging core is positioned in the imaging window region corresponding to the first tube during the imaging operation of the fluorescence lifetime imaging catheter for the internal body tissue.

18. The fluorescence lifetime imaging catheter of claim 12, wherein the fluorescence lifetime imaging catheter calculates a calibration value of the fluorescence lifetime imaging catheter using a predefined fluorescence lifetime value of an autofluorescence of the second tube before the imaging operation for the internal body tissue.

19. The fluorescence lifetime imaging catheter of claim 18, wherein the focusing device of the imaging core is moved from a first position in the imaging window region corresponding to the first tube to a second position in the distal tip region corresponding to the second tube to calculate the calibration value of the fluorescence lifetime imaging catheter.

20. The fluorescence lifetime imaging catheter of claim 18, wherein the calibration value includes a first calibration value calculated based on first fluorescence intensity information corresponding to the intensity of the fluorescence acquired while the focusing device is provided at a first position of the imaging window region and second fluorescence intensity information corresponding to the intensity of the fluorescence acquired while the focusing device is provided at a second position of the distal tip region.

21. The fluorescence lifetime imaging catheter of claim 18, wherein the calibration value includes a second calibration value calculated based on first fluorescence lifetime information corresponding to a lifetime of fluorescence acquired while the focusing device is provided at a first position of the imaging window region and second fluorescence lifetime information corresponding to a lifetime of fluorescence acquired while the focusing device is provided at a second position of the distal tip region.

22. The fluorescence lifetime imaging catheter of claim 20 or claim 21, wherein the fluorescence lifetime imaging catheter calibrates fluorescence intensity and fluorescence lifetime acquired by the fluorescence lifetime imaging catheter by applying the calibration value to fluorescence information acquired while the focusing device is provided at the first position during the imaging operation of the fluorescence lifetime imaging catheter for the internal body tissue.
